# EUROPEAN PATENT SPECIFICATION

(11) **EP 1 441 651 B1**
(45) Date of publication and mention of the grant of the patent: **31.08.2005**
(21) Application number: 02733091.9
(22) Date of filing: 31.05.2002
(51) Int. Cl.: A61B 17/16, B25B 23/00

(54) **TOOL BIT DRIVE SHAFT CONNECTION**
SCHNEIDEINSATZ-ANTRIEBSWELLE VERBINDUNG
ACCOUPLEMENT OUTIL RAPPORTE / TIGE D'ENTRAINEMENT

(30) Priority: 05.11.2001 US 338718 P
(43) Date of publication of application: 04.08.2004
(73) Proprietor: PRECIMED S.A., 2534 Orvin (CH)
(72) Inventor: WHITE, Patrick, Michel, Downingtown, PA 18358 (US)
(74) Representative: Grosfillier, Philippe
(86) International application number: PCT/IB2002/001950
(87) International publication number: WO 2003/039379

(56) References cited:
- EP-A- 0 444 776
- US-A- 5 277 435
- US-A- 5 797 918

## Description

### Technical Field

This invention relates generally to the connection of tool bits to superelastic flexible tubular drive shafts with particular emphasis on quick long-life connect/disconnect apparatus for mounting surgical tool bits on superelastically flexible, preferably nickel-titanium alloy, tubular drive shafts in surgical environments.

### Background Art

Connector devices for rotatable tubular drive members and tool bits, particularly for surgical applications, are known with one such connector being disclosed in United States patent 5,203,595.

The '595 apparatus is understood to have enjoyed a modest degree of commercial success. However, there are shortcomings in the '595 apparatus. Specifically, in a surgical environment, sterility of the equipment is an absolute requirement. Assuring a sterile environment means that all equipment introduced into the patient's body or in proximity to the patient must be sterile. To assure sterile conditions, such equipment is autoclaved after each surgical procedure.

Autoclaving and the high temperatures and humidities associated therewith results in the flexible material, namely polypropylene or other suitable material as recited at column 4, line 53 of '595, becoming brittle and failure prone long before the coupling device has otherwise worn out.

Additionally, the wedging action to which the thermoplastic is subjected in '595 contributes to rapid deterioration of the flexible polypropylene material. Moreover, the sliding engagement of the major mating members of '595 results in continuous wearing of the thermoplastic material.

Accordingly, the need remains for highly reliable, reusable connectors for tubular drive apparatus in a surgical environment. Increasing the useful life of the connector is highly desirable in order to reduce the skyrocketing costs associated with surgery.

A further coupling apparatus according is the preamble of claim 1 is known from US-A-5,797,918.

### Disclosure of the Invention

This invention provides coupling apparatus for co-axially connecting a according is claim 1. rotatable tool bit to a tubular drive member

Further preferred aspects of the invention are described in the dependent claims.

### Brief Description of Drawings

Figure 1 is an exploded perspective view of a long-life tubular drive shaft assembly for use with a quick connect tool bit, all in accordance with the preferred embodiment of the apparatus aspects of the invention, with two tool bits having different bit sizes being shown and each being ready to be mounted on the drive shaft assembly.
Figure 2 is a perspective view of the tubular drive shaft assembly of Figure 1 in an assembled condition, with no tool bit connected to the drive shaft assembly.
Figure 3 is a side view of a male drive fitting portion of the tubular drive shaft assembly illustrated in Figures 1 and 2, as indicated by dotted circle 3 in Figure 2.
Figure 4 is a perspective view of the drive shaft assembly of Figures 1 and 2 in an assembled condition with a tool bit connected to the drive shaft assembly.
Figure 5 is an exploded perspective view of a tubular drive shaft assembly for use with a quick connect tool bit in accordance with an alternate embodiment of the apparatus aspects of the invention, with a tool bit shown separate from but ready to be mounted on the drive shaft assembly.
Figure 6 is a perspective view of the tubular drive shaft assembly of Figure 5 in an assembled condition, with a quick connect tool bit shown separate from but ready to be mounted on the drive shaft assembly.
Figure 7 is a perspective view of the tubular drive shaft of Figures 5 and 6 with a tool bit mounted on the drive shaft assembly.

### Best Mode for Carrying Out the Invention

This invention is premised on the inventor's discovery that superelastic alloys, particularly superelastic nickel-titanium alloys, may be used effectively to create long-life quick disconnect detent action connecting devices in which the required elastic or snap-action characteristic of the detent is induced by creating internal stresses within the alloy member, resulting in material phase change and superelasticity, where the stresses are induced by flexure of the superelastic member by a second member as and when the two members are assembled together in the desired connection configuration.

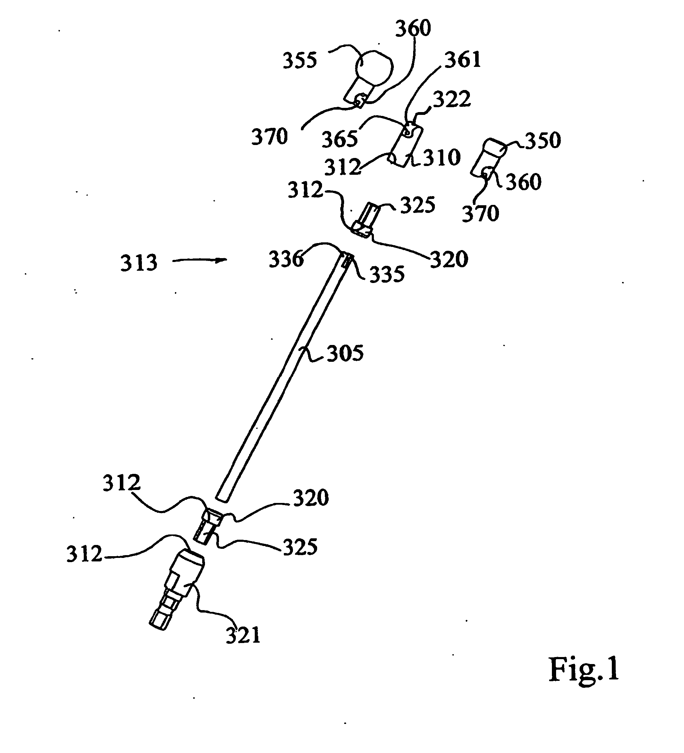

Referring to the drawings in general and to Figure 1 in particular, which is an exploded view of a tubular drive shaft assembly for use with a quick connect tool bit in accordance with the preferred embodiment of the apparatus aspects of the invention, the drive shaft assembly is designated generally 313 and includes an axially elongated hollow tubular shaft 305 preferably fabricated from suitable superelastic nickel-titanium alloy preferably of the type generally identified by the trademark Nitinol. Tubular shaft 305 at one end thereof has a relief groove or slot 335 formed therein serving to define two preferably axially extending tongues 336 which are superelastically flexible in the radial direction upon application of suitable stress, as a result of the Nitinol alloy stress-induced phase change and resulting superelasticity characteristic

Drive shaft assembly 313 further includes an annularly configured connector 320 which includes an axially elongated collet portion 325 extending from an annular base portion of connector 320. Collet portion 325 is fabricated from suitable flexible material, preferably stainless steel, such that collet portion 325 may radially bend or flex upon application of radially inward force thereto. Connector 320 with its annular base portion is sized to receive shaft 305 in reasonably tight but sliding engagement.

During operation, tubular shaft 305 is rotated in order to rotatably drive a surgical fitting to which shaft 305 is connected in part via connector 320, as described in greater detail below.

Located at an end of tubular shaft 305 opposite that at which tongues 336 are formed is a second connector 320 including a radially flexing collet portion 325 and an annular base portion, with such connector receiving a remaining end of tubular shaft 305 therewithin.

A power drive fitting 321 is provided for transmitting torque to shaft 305 from an external source; power drive fitting 321 grips tubular shaft 305 as a result of radially outward superelastic phase change deformation of shaft 305 against collet portion 325, in reaction to radially inward forces applied to tubular shaft 305 by collet 325. Collet portion 325 extends from a base portion of flexible connector 320 which fits together with power drive fitting 321, with these components preferably being welded or otherwise bonded together as indicated by weld line(s) 312 in Figures 1, 2 and 4.

Still referring to Figure 1, a tool drive fitting for transmitting torque from shaft 305 to a driven tool bit, such as either of the small and large tool bits respectively designated 350, 355 in Figure 1, is designated generally 310. In this regard, provision of a kit or other collection of a larger plurality of tool bits, of differing sizes and/or as replenishments one for another, is envisioned as an aspect of this invention. Tool drive fitting 310 is preferably generally cylindrical in form and in any event desirably has a continuously curved circumferential outer surface which may, as an alternative, be of frusto-conical or other curved configuration. Tool drive fitting 310 has an open center for axial passage therethrough of a guide wire along which drive shaft assembly 313 travels, using the wire as a guide during a surgical procedure. The hollow interior of tool drive fitting 310 additionally accommodates the preferably radially flexing collet portion 325 of connector 320. An opening or canulation 322 is provided preferably along the axis of tool drive fitting 310 for passage of the surgical guide wire out of tool drive fitting 310.

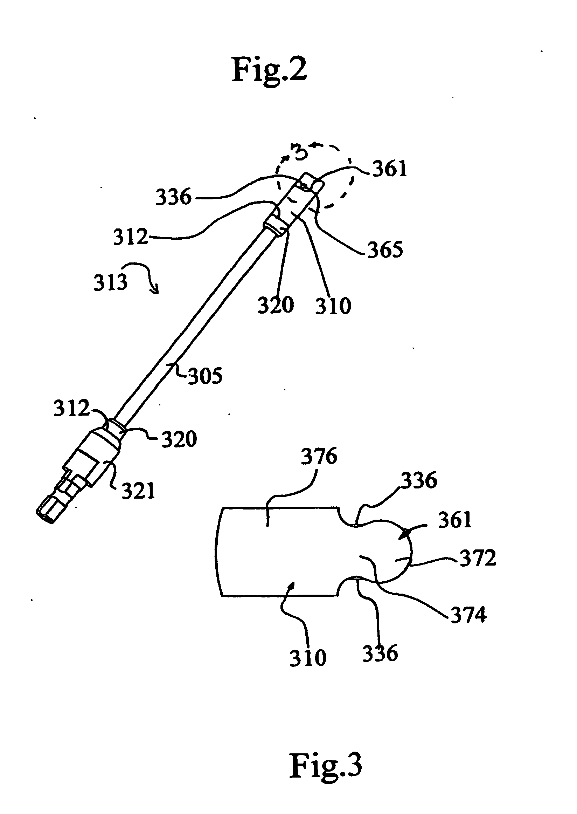

Referring to Figures 1 and 3, a mating tip portion of tool drive fitting 310 is referred to as a male tool bit interface 361 and preferably includes a bulbous portion 372 connecting to a main, preferably cylindrically configured, body portion 376 of tool drive fitting 310 via a neck portion 374, which is of smaller cross-section than bulbous portion 372 as readily apparent fran Figure 3. Cylindrical body portion of tool drive fitting 310 is designated 376 in Figure 3.

Exterior surfaces of bulbous portion 372 and neck portion 374 are curved and connect together in a continuous fashion so that bulbous portion 372 and neck portion 374 preferably form a structure like that found in interlocking jigsaw puzzle pieces. Cylindrical body portion 376 is preferably of larger cross-section, as illustrated in Figure 3, than is bulbous portion 372. The central passageway through tool drive fitting 310, for passage of the guide wire and for receipt of the radially flexing collet portion 325 of connector 320, is not illustrated in Figure 3 to enhance drawing clarity.

Tool drive fitting 310 further includes at least one preferably radially facing aperture formed in neck portion 374 and providing communication between the curved, external surface of neck portion 374 and the central axially extending passageway within tool drive fitting 310. One such aperture, designated generally 365, is shown in Figures 1 and 2.

When the drive shaft assembly of Figures 1 and 2 is assembled, the end of shaft 305 at which radially reactively elastically deformable tongues 336 are located is inserted through connector 320 sufficiently far that the axial extremities of tongues 336 extend out of a distal end of connector 320, beyond the axial extremity of collet 325. Tool drive fitting 310 is then slid over collet portion 325 with the elastically flexing canulation of collet portion 325 pressing radially inwardly causing stresses in nickel-titanium alloy shaft 305, resulting in stress-produced phase-change induced superelasticity and resulting reactive force exerted radially outwardly by tube 305, retaining tool drive fitting 310 in position. Tubular shaft 305 is further inserted until tongues 336 reach radially opening windows or apertures 365 at which the radially outward bias or superelastic spring action of tongues 336, resulting from the radially inward flexing of tongues 336 upon passage through fitting 310 and the consequent phase change and adoption of a superelastically deformable characteristic by the nickel-titanium alloy from which shaft 305 is fabricated, causes tongues 336 effectively to detent radially outwardly into windows or apertures 365 and protrude slightly therefrom in the radial direction as illustrated in Figure 3.

Tool bits for use in accordance with the drive shaft assembly 313 have tool drive fitting 310 with male tool bit interface 361 configured as shown and described above respecting Figures 1, 2 and 3. Each tool bit 350, 355 includes a female tool bit interface surface 360 configured for complemental facing contact with exteriorly facing surfaces of bulbous portion 372 and neck portion 374 of tool drive fitting 310. Formed within each female tool bit interface surface 360 is a recess 370 providing a detenting receptacle for receiving one of radially reactively superelastically flexing tongues 336 when tool bit interface 360 is in complemental contact with external surfaces of reck portion 374 and bulbous portion 372 of tool drive fitting 310. When this occurs, resulting stressinduced superelastic radial bias of tongues 336 causes those tongues to engage respective apertures 370, thereby providing a detenting action retaining tool drive fitting 310 in mating contact and coupled connection with a selected tool bit 350 or 355.

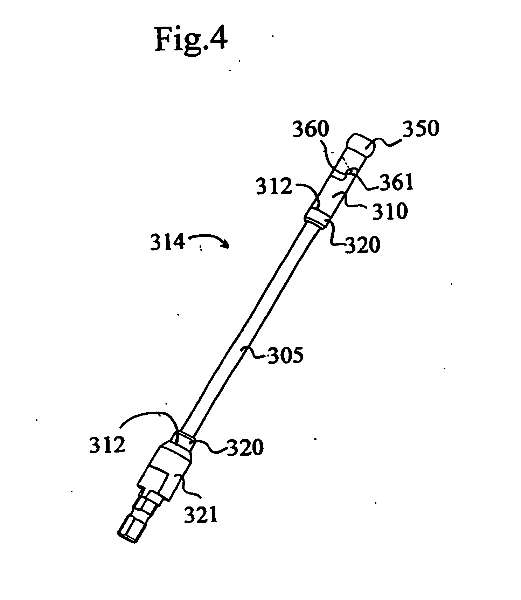

Such mated, coupled configuration of a selected small tool bit 350 with drive shaft assembly 313 is illustrated in Figure 4 where the assembly of drive shaft 313 and tool bit 350 is designated as a drive shaft and tool bit assembly 314.

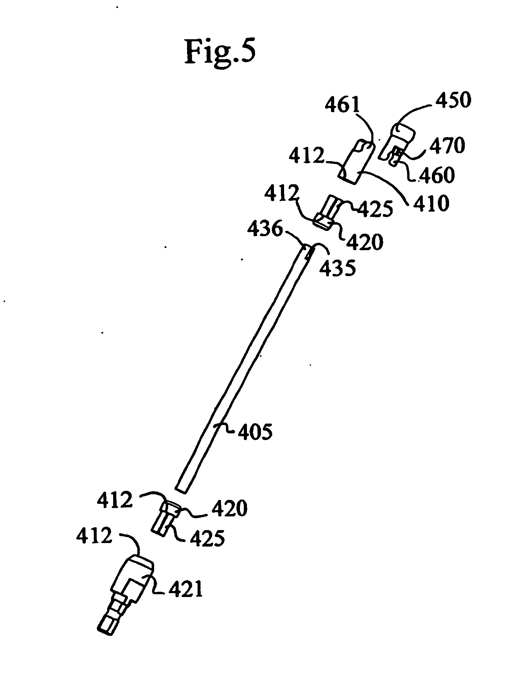

Figures 5, 6 and 7 disclose an alternate embodiment of a tubular long-life drive shaft assembly in accordance with apparatus aspects of the invention. Referring specifically to Figure 5, shaft 405 is analogous to shaft 305 illustrated in Figures 1, 2 and 4. Similarly, radially flexible connector 420 having a radially flexible collet portion 425 and a power drive fitting all are analogous to corresponding components numbered 320, 325 and 321 in Figures 1, 2 and 4.

Still referring to Figure 5, shaft 405 preferably includes a relief groove or slit 435 thereby defining a pair of stress-induced radially superelastically flexible tongues 436 which preferably correspond generally to tongues 336 illustrated in Figures 1, 2 and 4.

Still referring to Figure 5, a tool drive fitting 410 for transmitting torque from shaft 405 to a driven tool includes male tool bit interface which is preferably configured having an external surface preferably in the shape of a jigsaw puzzle piece configuration such as that defined by bulbous portion 372 and neck 374 in Figure 3.

However, male tool bit interface 461 preferably does not include radially facing apertures or windows such as 365 illustrated in Figures 1 and 2. Instead, in the alternate embodiment illustrated in Figures 5 through 7, radially superelastically reactively flexing tongues 436 preferably extend axially from tool drive fitting 410, exiting from tool drive fitting 410 preferably via an aperture defining the opening for the central passageway through which the guide wire extends when the apparatus of Figures 5 through 7 is used in a surgical environment.

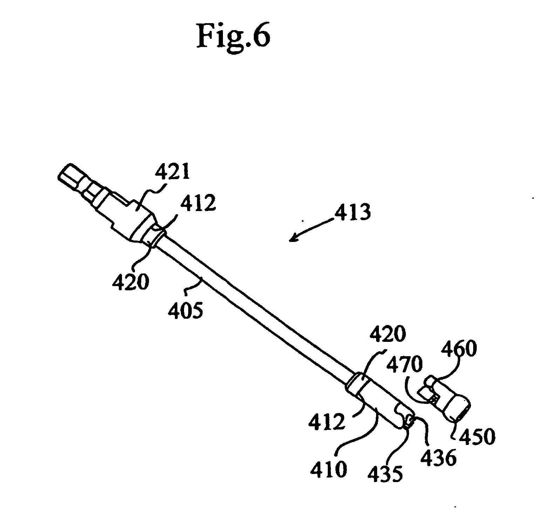

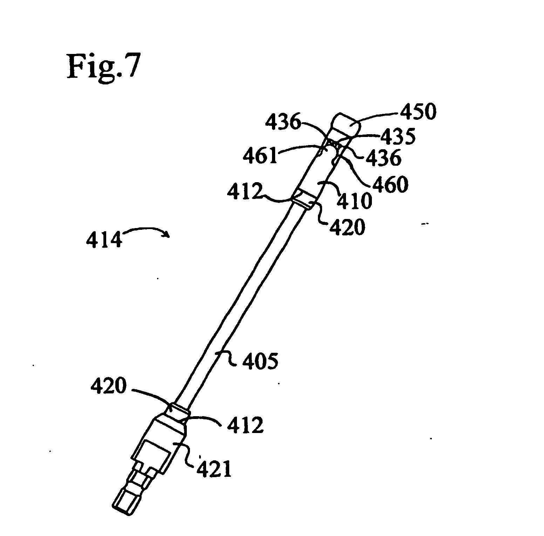

A tool bit 450 which is adapted for use with drive shaft assembly 413 and specifically with tool drive fitting 410 preferably includes a female mating portion shaped for complemental contact and interlocking engagement with the male jigsaw puzzle piece shape of male tool bit interface 461. An annular recess is formed within female tool bit interface surface 460 by making an axial bore. This axial bore is preferably of diameter slightly less than the minimum cross-sectional dimension of the neck portion of male tool bit interface surface 461 corresponding to neck portion 374 in Figure 3. As a result, the axial bore in small tool bit interface 460 provides an annular recess for detenting action whereby when small tool bit 450 is assembled over tool drive fitting 410, as illustrated generally in Figure 6 and specifically in Figure 7, axially extending radially superelastically reactively flexing tongues 436 flex radially inwardly and resultantly spring radially outwardly, due to their superelastic characteristic resulting from the radially inward flexure, contacting the annular surface of the axial bore as illustrated in Figure 7 thereby retaining small tool bit 450 in mating engagement with tool drive fitting 410.

The coupling apparatus of the invention is preferably designed so that resulting stresses and flexures of the superelastic material parts are always within the envelope of superelastic behavior. As a result, there is no fatigue or wear-out problem with the coupling apparatus of the invention. Additionally, the nickel-titanium superelastic alloys which are preferredfor use in this invention are very hard - much harder than stainless steels, from which surgical cutting tool bits of the type to be typically used with this invention are made. Hence, over time the tool bits, which wear as a result of use, additionally wear at the locations of contact with the superelastic alloy parts, while the superelastic parts, being harder than stainless steel, do not wear at such areas of contact. As a result, the coupling apparatus according to the invention should exhibit nearly infinite life.

Multiple variations and modifications are possible in the embodiments of the invention described here. Although certain illustrative embodiments of the invention have been shown and described here, a wide range of modifications, changes, and substitutions is contemplated in the foregoing disclosure. Accordingly, it is appropriate that the foregoing description be construed broadly and understood as being given by way of illustration and example only, the scope of the invention being limited only by the appended claims.

## Claims

1. Coupling apparatus for coaxially connecting a rotatable tool bit (350, 355, 450) to a tubular drive member (305, 405), **characterized in that** :
a) it comprises a first tubular part (310, 410) having a male member extending axially therefrom including a first mating interface surface (361, 461);
b) the tool bit (350, 355, 450) has an axial female bore forming a receptacle for receiving the first tubular part (310, 410) therewithin including a second mating interface surface (360, 460) formed within the female bore and shaped for receptive complemental facing with the first mating interface surface (361, 461) of the first tubular part upon insertion of the first tubular part into the receptacle, with a recess (370, 470) being formed in the second mating interface surface (360, 460);
c) the tubular drive member (305, 405) has at least one axially elongated radially flexible tongue (336, 436) extending from an end of the tubular drive member (305, 405) to which the tool bit (350, 355, 450) is to be secured into the recess (370, 470) to provide radial detenting attachment of the tool bit to the tubular drive member via superelastic radial bias of the tongue (336, 436) induced by radially opposite flexure of the tongue upon passage thereof into the first tubular part (310, 410) to reach the recess (370, 470).

2. Coupling apparatus according to claim 1, **characterized in that** the recess (470) is an axial annular recess.

3. Coupling apparatus according to claim 1, **characterized in that**
a) the first tubular part (310) is provided with a passageway (365) extending from an axially open center of the first tubular part and opening onto the mating interface surface (361);
b) the recess (370) communicates with the passageway (365) when the tool bit (350, 355) and the first tubular part (310) are engaged with the mating interface surfaces (360; 361) complementally facing one another;
c) the axially elongated radially flexible tongue (336) of the tubular drive member (305) protrudes through the passageway (365) and into the recess (370) to provide detenting attachment of the tool bit (350, 355)to the tubular drive member.

4. Coupling apparatus according to claim 1, **characterized in that**:
a) the first tubular part (310) has a jigsaw-puzzle shaped member (310, 361, 372, 374, 376) extending axially therefrom and including a bulbous extremity portion (372) connected to a body (376) of the first tubular part by a neck portion (374), exteriors of the neck and bulbous portions together defining a first continuously curved mating interface surface which is convex about the bulbous portion and concave about the neck portion, with a radial passageway (365) extending from an axially open center of the first tubular part (310) through the neck portion (374) and opening onto the concave portion of the continuously curved mating interface surface;
b) it comprises a collar-like connector (320) having an annular base portion and a radially flexing collet (325) extending axially therefrom;
c) the tool bit (350, 355) axial bore receives and radially deforms the collet (325) against the tubular drive member (305) to secure the connector (320) to the tubular drive member via superelastic radially outward reactive bias of the tubular drive member against the collet induced by radially inward flexure of the tubular drive member (305) by the collet (325), with an axially oriented jigsaw-puzzle (360) shaped receptacle having a second continuously curved mating interface surface formed within the bore and shaped for receptive complemental contact with the first continuously curved mating interface surface (361) of the first tubular part (320) upon axial insertion of the first part into the receptacle, the radial recess (370) being formed in a convex portion of the second continuously curved mating interface surface (361) and communicating with the radial passageway (365) when the tool bit and the first tubular part (320) are engaged with the mating interface surfaces facingly complementally contacting one another;
d) the axially elongated radially elastically deformable tongue (336) of the tubular drive member (305) protrudes through the radial passageway (365) and into the radial recess (370)to provide detenting attachment of the tool bit to the rotatable tubular drive member.

5. Coupling apparatus according to claim 2, **characterized in that**
a) the first tubular part (410) male member is a jigsaw-puzzle shaped member (410,461) extending axially therefrom and including a bulbous extremity portion connected to a body of the first tubular part by a neck portion,exteriors of the neck and bulbous portions together defining a first continuously curved mating interface surface which is convex about the bulbous portion and concave about the neck portion;
b) it comprises a collar-like connector (420) having an annular base portion and a radially deformable collet (425) extending axially therefrom;
c) the tool bit (450) axial bore receives and radially flexes the collet (425) against the tubular drive member (405) to secure the collar-like connector (420) to the tubular drive member (405) via superelastic radially outward reactive bias of the tubular drive member against the collet induced by radially inward flexure of the tubular drive member by the collet, with an axially oriented jigsaw-puzzle shaped receptacle the second continuously curved mating interface surface (460) formed within the bore and shaped for receptive complemental contact with the first continuously curved mating interface surface (461) of the first tubular part (410) upon axial insertion of the first tubular part into the receptacle.;
d) the at least one axially elongated radially elastically deformable tongue (436) extends into the axial annular recess (470) to provide detenting attachment of the tool bit (450) to the rotatable drive member (405) via reactive superelastic radial bias of the tongue induced by radially opposite flexure of the tongue upon passage thereof into the first tubular part to reach the axial radial recess (470).

## Patentansprüche

1. Kupplungsapparat zur koaxialen Verbindung eines rotierbaren Werkzeugeinsatzes (350, 355, 450) mit einem rohrförmigen Antriebselement (305, 405), **dadurch gekennzeichnet, dass** :
a) der Apparat ein erstes rohrförmiges Teil (310, 410) mit einem vorstehenden Bereich aufweist, der sich in Axialrichtung vom rohrförmigen Teil erstreckt und eine erste Verbindungs-Passfläche (361, 461) besitzt;
b) der Werkzeugeinsatz (350, 355, 450) eine axiale einspringende Bohrung aufweist, welche eine Aufnahme für das erste rohrförmige Teil (310, 410) bildet und in der einspringenden Bohrung eine zweite Verbindungs-Passfläche (360, 460) besitzt, welche zur aufnehmenden komplementären Anlage an die erste Verbindungs-Passfläche (361, 461) des ersten rohrförmigen Teils geformt ist, wobei diese Anlage beim Einsetzen des ersten rohrförmigen Teils in die Aufnahme eintritt, und wobei in die zweite Verbindungs-Passfläche (360, 460) eine Vertiefung (370, 470) eingeformt ist;
c) das rohrförmige Antriebselement (305, 405) mindestens eine axial gestreckte und radial flexible Zunge (336, 436) aufweist, die sich von einem Ende des rohrförmigen Antriebselements (305, 405), an welchem der Werkzeugeinsatz (350, 355, 450) zu befestigen ist, in die Vertiefung (370, 470) erstreckt, um ein radial lösbares Anbringen des Werkzeugeinsatzes am rohrförmigen Antriebselement über eine superelastische Verspannung der Zunge (336, 436) durch radial entgegengesetztes Verbiegen der Zunge bei deren Eintreten in das erste rohrförmige Teil (310, 410) bis zum Erreichen der Vertiefung (370, 470) zu bewerkstelligen.

2. Kupplungsapparat nach Anspruch 1, **dadurch gekennzeichnet, dass** die Vertiefung (470) eine axiale ringförmige Ausnehmung ist.

3. Kupplungsapparat nach Anspruch 1, **dadurch gekennzeichnet, dass**
a) das erste rohrförmige Teil (310) mit einem Durchgang (365) versehen ist, der sich von einem axial offenen Zentrum des ersten rohrförmigen Teils aus erstreckt und sich in die Verbindungs-Passfläche (361) öffnet;
b) die Vertiefung (370) mit dem Durchgang (365) in Verbindung steht, wenn der Werkzeugeinsatz (350, 355) und das erste rohrförmige Teil (310) mit den Verbindungs-Passflächen (360; 361) in Eingriff sind, die sich komplementär gegenüberstehen;
c) die axial gestreckte und radial flexible Zunge (336) des rohrförmigen Antriebselements (305) durch den Durchgang (365) in die Vertiefung (370) ragt, um ein lösbares Anbringen des Werkzeugeinsatzes (350, 355) am rohrförmigen Antriebselement zu schaffen.

4. Kupplungsapparat nach Anspruch 1, **dadurch gekennzeichnet, dass**
a) das erste rohrförmige Teil (310) ein puzzleteilförmiges Element (310, 361, 372, 374, 376) aufweist, das sich axial vom rohrförmigen Teil erstreckt und einen kugeligen Endbereich (372) besitzt, der mit einem Körper (376) des ersten rohrförmigen Teils über einen Steg (374) verbunden ist, wobei das Äussere des Stegs und des kugeligen Teils zusammen eine erste kontinuierlich gekrümmte Verbindungs-Passfläche bilden, die am kugeligen Bereich konvex und am Steg konkav ist, wobei sich ein radialer Durchlass (365) von einem axial offenen Zentrum des ersten rohrförmigen Teils (310) durch den Steg (374) hindurch erstreckt und sich in den konkaven Bereich der kontinuierlich gekrümmten Verbindungs-Passfläche öffnet;
b) er ein manschettenähnliches Verbindungsstück (320) mit einem ringförmigen Grundbereich und einer axial anschliessenden radial nachgiebigen Hülse (325) aufweist;
c) die Axialbohrung des Werkzeugeinsatzes (350, 355) die Hülse (325) aufnimmt und sie gegen das rohrförmige Antriebselement (305) radial verformt, um das Verbindungsstück (320) am rohrförmigen Antriebselement über eine radial nach aussen gerichtete superelastische Verspannung des rohrförmigen Antriebselements gegen die Hülse, verursacht durch eine radial nach innen gerichtete Beugung des rohrförmigen Antriebselements (305) durch die Hülse (305), zu befestigen, wobei ein axial ausgerichtetes puzzleteilförmiges Aufnahmeelement (360), das eine zweite kontinuierlich gekrümmte Verbindungs-Passfläche aufweist, welche im Inneren der Bohrung ausgeformt ist und zur komplementär geformten Anlage an die erste kontinuierlich gekrümmte Verbindungs-Passfläche (361) des ersten rohrförmigen Teils (320) beim axialen Einführen des ersten Teils in die Aufnahme ausgebildet ist, und wobei die radiale Vertiefung (370) in einem konvexen Bereich der zweiten kontinuierlich gekrümmten Verbindungs-Passfläche (361) angebracht ist und mit dem radialen Durchlass (365) in Verbindung steht, wenn der Werkzeugeinsatz mit dem ersten rohrförmigen Teil (310) über die Verbindungs-Passflächen mit einer gegenseitig komplementären Anlage verbunden ist;
d) die axial gestreckte und radial elastisch verformbare Zunge (336) des rohrförmigen Antriebselements (305) durch den radialen Durchlass (365) in die radiale Vertiefung (370) ragt, um eine lösbare Befestigung des Werkzeugeinsatzes am rotierbaren rohrförmigen Antriebselement zu schaffen.

5. Kupplungsapparat nach Anspruch 2, **dadurch gekennzeichnet, dass**
a) der vorstehende Bereich des ersten rohrförmigen Teils (410) ein puzzleteilförmiges Element (410, 461) ist, welches sich axial vom rohrförmigen Teil erstreckt und einen kugeligen Endbereich (372) besitzt, der mit einem Körper des ersten rohrförmigen Teils über einen Steg verbunden ist, wobei das Äussere des Stegs und des kugeligen Teils zusammen eine erste kontinuierlich gekrümmte Verbindungs-Passfläche bilden, die am kugeligen Bereich konvex und am Steg konkav ist;
b) er ein manschettenähnliches Verbindungsstück (420) mit einem ringförmigen Grundbereich und einer axial anschliessenden radial nachgiebigen Hülse (425) aufweist;
c) die Axialbohrung des Werkzeugeinsatzes (450) die Hülse (425) aufnimmt und sie gegen das rohrförmige Antriebselement (405) radial verformt, um das Verbindungsstück (420) am rohrförmigen Antriebselement (405) über eine radial nach aussen gerichtete reaktive superelastische Verspannung des rohrförmigen Antriebselements gegen die Hülse, verursacht von der Hülse durch eine radial nach innen gerichtete Beugung des rohrförmigen Antriebselements, zu befestigen, wobei ein axial ausgerichtetes puzzleteilförmiges Aufnahmeelement, das die zweite kontinuierlich gekrümmte Verbindungs-Passfläche (460) aufweist, welche im Inneren der Bohrung ausgeformt ist und zur komplementär geformten Anlage an die erste kontinuierlich gekrümmte Verbindungs-Passfläche (461) des ersten rohrförmigen Teils (410) beim axialen Einführen des ersten Teils in die Aufnahme ausgebildet ist;
d) die mindestens eine axial gestreckte und radial elastisch verformbare Zunge (436) in die radiale Vertiefung (470) ragt, um eine lösbare Befestigung des Werkzeugeinsatzes (450) am rotierbaren rohrförmigen Antriebselement (405) über eine reaktive superelastische Verspannung der Zunge durch radial entgegengesetztes Verbiegen der Zunge bei deren Eintreten in das erste rohrförmige Teil bis zum Erreichen der radialen ringförmigen Vertiefung (470) zu schaffen.

## Revendications

1. Accouplement pour une connexion coaxiale d'une partie d'outil rotatif (350, 355, 450) à un élément tubulaire d'entraînement (305, 405), **caractérisé en ce que** :
a) il comprend une première pièce tubulaire (310, 410) comportant un élément mâle s'étendant axialement à partir de la pièce tubulaire et présentant une première surface de jonction appariée (361, 461);
b) la partie d'outil (350, 355, 450) présente un alésage femelle axial formant un récipient de réception pour la première pièce tubulaire (310, 410), l'alésage femelle comprenant en son intérieur une deuxième surface de jonction appariée (360, 460) dont la forme constitue un complément réceptif à la première surface de jonction appariée (361, 461) de la première pièce tubulaire lors de l'introduction de la première pièce tubulaire dans le récipient, un évidement (370, 470) étant formé dans la deuxième surface de jonction appariée (360, 460);
c) l'élément tubulaire d'entraînement (305, 405) comporte au moins une languette axialement allongée et radialement flexible (336, 436) s'étendant à partir d'une extrémité de l'élément tubulaire d'entraînement (305, 405) auquel la partie d'outil (350, 355, 450) doit être fixée, dans l'évidement (370, 470) afin de fournir une attache radialement amovible de la partie d'outil à l'élément tubulaire d'entraînement via une contrainte radiale super-élastique de la languette (336, 436), causée par une flexion radialement opposée de la languette lors de son passage dans la première pièce tubulaire (310, 410) pour atteindre l'évidement (370, 470).

2. Accouplement selon la revendication 1, **caractérisé en ce que** l'évidement (470) est un évidement axial annulaire.

3. Accouplement selon la revendication 1, **caractérisé en ce que**
a) la première pièce tubulaire (310) est dotée d'un passage (365) s'étendant à partir d'un centre axialement ouvert de la première pièce tubulaire et s'ouvrant dans la première surface de jonction appariée (361);
b) l'évidement (370) communique avec le passage (365) lorsque la partie d'outil (350, 355) et la première pièce tubulaire (310) sont engagés avec les surface de jonction appariées (360; 361) et se juxtaposent de manière complémentaire;
c) la languette axialement allongée et radialement flexible (336) de l'élément tubulaire d'entraînement (305) s'étend à travers le passage (365) et dans l'évidement (370) pour fournir une fixation amovible de la partie d'outil (350, 355) sur l'élément tubulaire d'entraînement.

4. Accouplement selon la revendication 1, **caractérisé en ce que** :
a) la première pièce tubulaire (310) comporte une partie en forme de pièce de puzzle (310, 361, 372, 374, 376) qui s'étend axialement à partir de la pièce tubulaire et comprend une extrémité bulbeuse (372) connectée à un corps (376) de la première pièce tubulaire par l'intermédiaire d'une partie d'entretoise (374), les contours de l'entretoise et du bulbe définissant ensemble une première surface de jonction appariée à courbure continue qui est convexe autour du bulbe et concave autour de l'entretoise, un passage radial (365) s'étendant à partir d'un centre axialement ouvert de la première pièce tubulaire (310) à travers l'entretoise (374) et s'ouvrant dans la partie concave de la surface de jonction appariée à courbure continue;
b) il comprend un connecteur en forme de manchon (320) présentant une partie de base annulaire et une douille radialement élastique (325) en extension axiale;
c) l'alésage axial de la partie d'outil (350, 355) reçoit la douille (325) et la déforme radialement contre l'élément tubulaire d'entraînement (305) afin de fixer solidement le connecteur (320) à l'élément tubulaire d'entraînement (305) via une contrainte superélastique réactive agissant radialement vers l'extérieur de l'élément tubulaire d'entraînement contre la douille, contrainte causée par la douille (325) qui engendre une flexion radiale de l'élément tubulaire d'entraînement (305) vers l'intérieur, un récipient axialement orienté en forme de pièce de puzzle (360) contenant une deuxième surface de jonction appariée à courbure continue dans son alésage et conformée pour fournir un contact complémentaire réceptif avec la première surface de jonction appariée à courbure continue (361) de la première pièce tubulaire (320) lors d'une introduction axiale de la première pièce tubulaire dans le récipient, l'évidement radial (370) étant formé dans une partie convexe de la deuxième surface de jonction appariée à courbure continue (361) et étant en communication avec le passage radial (365) lorsque la partie d'outil et la première pièce tubulaire (310) se sont engagés par un contact mutuel des surfaces de jonction appariées juxtaposées de façon complémentaire;
d) la languette axialement allongée et radialement déformable de manière élastique (336) de l'élément tubulaire d'entraînement (305) dépasse le passage radial (365) et entre dans l'évidement radial (370) pour fournir une fixation amovible de la partie d'outil à l'élément tubulaire d'entraînement rotatif.

5. Accouplement selon la revendication 2, **caractérisé en ce que** :
a) la partie mâle de la première pièce tubulaire (410) comporte une partie en forme de pièce de puzzle (410, 461) qui s'étend axialement à partir de la pièce tubulaire et comprend une extrémité bulbeuse connectée à un corps de la première pièce tubulaire par l'intermédiaire d'une partie d'entretoise, les contours de l'entretoise et du bulbe définissant ensemble une première surface de jonction appariée à courbure continue qui est convexe autour du bulbe et concave autour de l'entretoise,
b) il comprend un connecteur en forme de manchon (420) présentant une partie de base annulaire et une douille radialement déformable (425) en extension axiale;
c) l'alésage axial de la partie d'outil (450) reçoit la douille (425) et la déforme radialement contre l'élément tubulaire d'entraînement (405) afin de fixer solidement le connecteur (420) en forme de douille à l'élément tubulaire d'entraînement (405) via une contrainte superélastique réactive agissant radialement vers l'extérieur de l'élément tubulaire d'entraînement contre la douille, contrainte causée par la douille qui engendre une flexion radiale de l'élément tubulaire d'entraînement vers l'intérieur, un récipient axialement orienté en forme de pièce de puzzle et contenant une deuxième surface de jonction appariée à courbure continue (460) dans son alésage et conformée pour fournir un contact complémentaire réceptif avec la première surface de jonction appariée à courbure continue (461) de la première pièce tubulaire (410) sur une introduction axiale de la première pièce tubulaire dans le récipient;
d) la au moins une languette axialement allongée et radialement flexible (436) entre dans l'évidement axial annulaire (470) pour fournir une fixation amovible de la partie d'outil (450) à l'élément d'entraînement rotatif (405) via une contrainte superélastique réactive radiale de la languette causée par une flexion radialement opposée de la languette lors de son passage dans la première pièce tubulaire pour atteindre l'évidement axial annulaire (470).
